# EUROPEAN PATENT SPECIFICATION

(11) **EP 2 764 889 B1**
(45) Date of publication and mention of the grant of the patent: **13.07.2016**
(21) Application number: 14152632.7
(22) Date of filing: 27.01.2014
(51) Int. Cl.: A61N 1/372, A61N 1/378

(54) **Implantable medical device, medical system and method for data communication**
Implantierbare medizinische Vorrichtung, medizinisches System und Verfahren zur Datenkommunikation
Dispositif médical implantable, système médical et procédé de communication de données

(30) Priority: 07.02.2013 US 201361761707 P
(43) Date of publication of application: 13.08.2014
(73) Proprietor: BIOTRONIK SE & Co. KG, 12359 Berlin (DE)
(72) Inventor: Kibler, Andrew, Lake Oswego, OR Oregon 97035 (US); Moulder, J. Christopher, Portland, Oregon 97202 (US); Whittington, R. Hollis, Portland, Oregon 97202 (US)
(74) Representative: Galander, Marcus

(56) References cited:
- US-A1- 2008 294 062
- US-A1- 2009 228 071
- US-A1- 2010 125 315
- US-A1- 2011 125 214
- US-A1- 2011 288 615
- US-A1- 2012 004 708
- US-A1- 2012 029 323
- US-A1- 2012 101 545
- US-B2- 8 612 013

## Description

The present invention generally relates to implantable medical devices and data communication from such implantable medical device to an external device.

Implantable devices, in particular implantable medical devices, such as implantable therapy and/or monitoring devices including pacemakers, cardioverters and defibrillators or the like, may include data communication means to transmit data from the implantable medical device to a (body-)external device or vice versa.

A system for data communication with a medical device thus includes an implantable medical device and an external device such as a programmer.

A typical implantable medical device comprises a battery, a monitoring and/or therapy control unit, in some cases additionally one or more therapy units such as stimulation units and a memory for storing control program and/or data sensed by the implantable medical device. If the implantable medical device is a pacemaker or an implantable cardioverter/defibrillator (ICD), the therapy units comprise stimulation units for generating and delivering electric stimulation pulses to a patient's heart tissue (myocardium).

In order to transmit data sensed by the implantable medical device to an external device a telemetry unit may be provided. Typically the telemetry unit is configured to allow a bidirectional data communication, that is, the telemetry unit can transmit and receive data wirelessly.

US2008294062A1 discloses an ultra-low-power circuit for wireless neural recording and stimulation, wherein the circuit includes a neural amplifier with adaptive power biasing for use in multi-electrode arrays and a decoding and/or learning architecture.

US2009228071A1 describes techniques for communication between at least one leadborne device of an implantable lead and a medical device to which the lead is connected.

US2012004708A1 discloses an implantable medical device and an external base station system, wherein the external base station can provide a passive electric field to power the implant, or to charge its battery.

US2012029323A1 discloses an antenna for an implantable medical device for wirelessly communicating with another device.

US2010125315A1 discloses a method and system of providing therapy to a patient implanted with an array of electrodes.

Limited battery capacity of an implantable medical device calls for energy-efficient data communication. An implantable medical device with limited battery power requires a low power communication scheme in order to program it and to download acquired data. With extremely low power communication, more data can be transmitted more often.

Present communication schemes used for data communication by a telemetry unit involve either RF or magnetic communication. RF frequencies of ~400 or ~900 MHz or magnetic coupling in the 100s of kHz range require several mA of current to transmit and receive data. Such high current requirements are out of reach of devices with battery capacities of at most a few hundred mAh.

In addition, RF schemes require large antennas and magnetic coupling requires large transmit and receive coils for communication. The space available in miniaturized implants would not allow such large coils or antennas.

In view of the above, there is a need for a low power communication scheme that does not employ RF or magnetic coupling.

It is an object of the invention to provide an alternative implantable device and an alternative data communication method that result in little battery drain from the medical device's battery.

According to the invention, an implantable medical device is provided that comprises data communication means which include means for altering an oscillatory electric field imposed on body tissue surrounding the implantable medical device when the implantable medical device is in its implanted state. The data communications means include means of generating a second oscillatory electric field which is synchronized to a first oscillatory electric field imposed on body tissue surrounding the implantable medical device.

In a first embodiment, the means for altering an oscillatory electric field are configured to modulate an impedance of a volume of body tissue surrounding the implantable medical device when the implantable medical device is in its implanted state and within an oscillatory electric field.

In an alternative embodiment, the means for altering an oscillatory electric field comprise means for generating an oscillatory electric field that is phase-synchronized with an oscillatory electric field imposed on body tissue surrounding the implantable medical device when the implantable medical device is in its implanted state.

The means for generating a second oscillatory electric field comprise means for sensing the phase of a first oscillatory electric field by the implantable medical device, and means for the device to generate the second oscillatory electric field in a manner that is phase synchronized to the sensed first electric field while the medical device is in its implanted state.

The present invention uses modulation of impedance within an electric field to communicate between a small medical device implanted in the heart or body and an external programmer/communication device, which may also be implanted in the body. Alternatively, the invention may generate a small electric field to facilitate this same communication, thus modulating the electric field imparted on the implantable medical device. The external device uses cutaneous or implanted electrodes to impart an oscillatory electric field on the body that encompasses the medical device.

In the impedance modulation embodiment (first embodiment) of this invention, the medical device, within the field, alternates shorting and opening an internal connection between two electrodes on its surface to change the impedance across the space of the device. The change in impedance is sensed by the external device as changes in either current or voltage. The changes in voltage or current can be modulated to form a communication scheme to transmit data. The external device's electric field may be additionally used to transmit data to the implanted medical device.

In the electric field transmission embodiment (second embodiment) of this invention, the external device uses cutaneous or implanted electrodes to impart an oscillatory electric field on the body that encompasses the implanted medical device. The implanted device then monitors this field and generates a small electric field that is phase-synchronized with the external device's field in order to facilitate reception of this small field by the external device using a lock-in (phase-locked) amplifier. The external device's electric field may not be continuously generated, and may be additionally used to transmit data to the implanted medical device. The small changes in the body's electric field are sensed by the external device as changes in voltage or current and are demodulated to form a communications scheme from the implanted device to the external device.

The present invention allows an implantable medical device with limited battery supply the ability to transmit increased amount of data while using very little power. Data transmission is achieved by modulating an electric field imparted on the body by altering the impedance of the transmission medium and read by the receiver as changing voltage or current. Continuous medium rate data transmission can be achieved while using very little battery power.

Preferably, the implantable medical device according to the first embodiment further comprises switching means and at least two electrodes that are connected to the switching means so they can be electrically connected or disconnected, respectively, by the switching means, wherein the at least two electrodes are configured and arranged to cause a change of impedance when the implantable medical device is in its implanted state and the electrodes are connected or disconnected, respectively, by means of the switching means.

The switching means preferably are connected to a switch control that may be configured to sense an oscillatory electric field imposed on body tissue surrounding the implantable medical device when the implantable medical device is in its implanted state. Thus it is possible to synchronize connecting and disconnecting of the at least two implantable electrodes with the oscillatory electric field imposed on the body tissue surrounding or encompassing the implantable medical device.

To implement such synchronizing, it is preferred that the switch control comprises a phase-locked loop (PLL) and a frequency divider, wherein the phase-locked loop is configured to lock in a frequency of an oscillatory electric field imposed on body tissue surrounding the implantable medical device when the implantable medical device is in its implanted state. The frequency divider is connected to the phase-locked loop and is configured to divide a frequency signal put out by the phase-locked loop. Thus, the implantable medical device can generate a code that represents data to be transmitted from the implantable medical device to an external device, wherein the clock for such code is a fraction of the frequency of the oscillatory electric field imposed on the body tissue surrounding the implantable medical device. If the frequency of the divided signal is sufficiently low, on the order of 1:100, the need for the PLL would be eliminated. In this case the maximum decrease in efficiency due to lack of phase lock with the external electric field would be one percent, or one out of 100 cycles of the imparted field.

According to a further preferred embodiment, the switch control is connected to the at least two electrodes and is configured to sense an oscillatory electric field imposed on body tissue via the at least two electrodes. The switch control can comprise a band-pass filter, wherein the band-pass filter filters a signal fed to the phase-locked loop.

In an alternative embodiment, the means for altering an oscillatory electric field comprise field generating means for generating an oscillatory electric field that is phase-synchronized with an oscillatory electric field imposed on body tissue surrounding the implantable medical device when the implantable medical device is in its implanted state. An implantable medical device according to this embodiment may further comprise field generation control means that are operatively connected to the field generating means and are configured to control the field generating means in response to an oscillatory electric field imposed on body tissue surrounding the implantable medical device when the implantable medical device is in its implanted state.

The object of the invention is further achieved by a data communication system including an implantable medical device as described above and an external device that comprises or can be connected to at least two cutaneous or implanted electrodes. The external device further comprises external field generating means that are configured to generate an oscillatory electric field to be imposed across the implanted medical device via the at least two cutaneous or implanted electrodes. The external device further comprises means for sensing alterations of body impedance and/or an oscillatory electric field generated by the implantable medical device when the implantable medical device is in its implanted state.

The external device preferably comprises a lock-in amplifier, an AM demodulator for demodulating amplitude-modulated signals and an analog-to-digital converter, wherein the analog-to-digital converter is operatively connected to the AM demodulator and the lock-in amplifier, and wherein the analog-to-digital converter produces a signal that represents a signal transmitted by the implantable medical device.

The object of the invention is further achieved by a method of communicating data from an implantable medical device to an external device, said method comprising:
- imposing an oscillatory electric field in body tissue encompassing an implantable medical device,
- sensing the imposed oscillatory electric field by means of the implantable medical device,
- either:
   - altering the oscillatory electric field by means of the implantable medical device
      - by inducing an alternating change of impedance by means of the implantable medical device
      - or by generating a small oscillatory electric field by means o the implantable medical device,
      or
   - generating an oscillatory electric field which is phase synchronized to the imposed electric field by means of the implantable device
- sensing the change of impedance or the small oscillatory electric field generated by the implantable medical device, respectively, by means of an external device having or being connected to at least two cutaneous electrodes.

Preferably, the step of altering the oscillatory electric field by means of the implantable medical device is performed by means of at least two electrodes operatively connected or being part of the implantable medical device, and switching means operatively connected to the at least two electrodes, wherein the switching means connect and disconnect the at least two electrodes in an alternating manner according to a code-representing data to be transmitted from the implantable medical device to the external device, said alternating connecting and disconnecting causing a detectable change of impedance for the imposed oscillatory electric field.

The above and other aspects, features and advantages of the present invention will be more apparent from the following more particular description thereof, presented in conjunction with the following drawings, wherein:
- Figure 1:: is a representation of a communication system, comprising an implantable medical device in its implanted state and an external device;
- Figure 2:: is a more abstract representation of the system depicted in figure 1;
- Figure 3:: is a more detailed representation of an implantable device showing hose elements that are provided to implement the invention;
- Figure 4:: is a more detailed representation of an external device showing hose elements that are provided to implement the invention;
- Figure 5:: is circuit diagram for a simulation scenario for simulating impedance change based data communication;
- Figure 6:: is a plot illustrating a simulation result achieved by using the diagram depicted in figure 5.

The following description is of the best mode presently contemplated for carrying out the invention. This description is not to be taken in a limiting sense, but is made merely for the purpose of describing the general principles of the invention. The scope of the invention should be determined with reference to the claims.

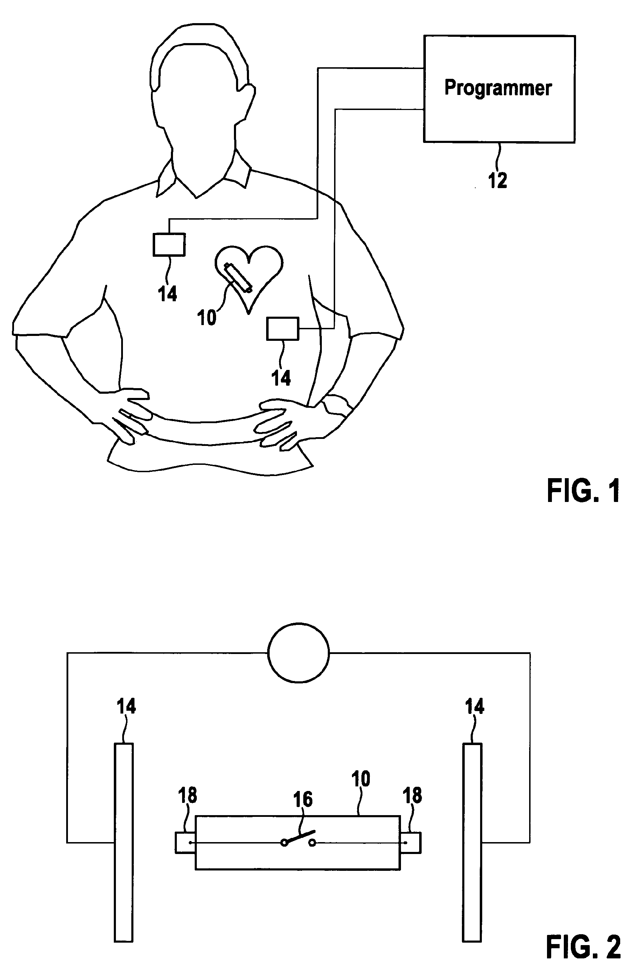

In one embodiment, utilizing an implanted pacemaker as an implantable medical device 10, a programmer/communication device as external device 12 uses cutaneous electrodes 14 placed on either side of the heart as shown in figure 1. The external device 12 induces an oscillating electric field between the electrodes at 100kHz - 1MHz at a known voltage or current. The medical device implanted in the heart is between the two electrodes.

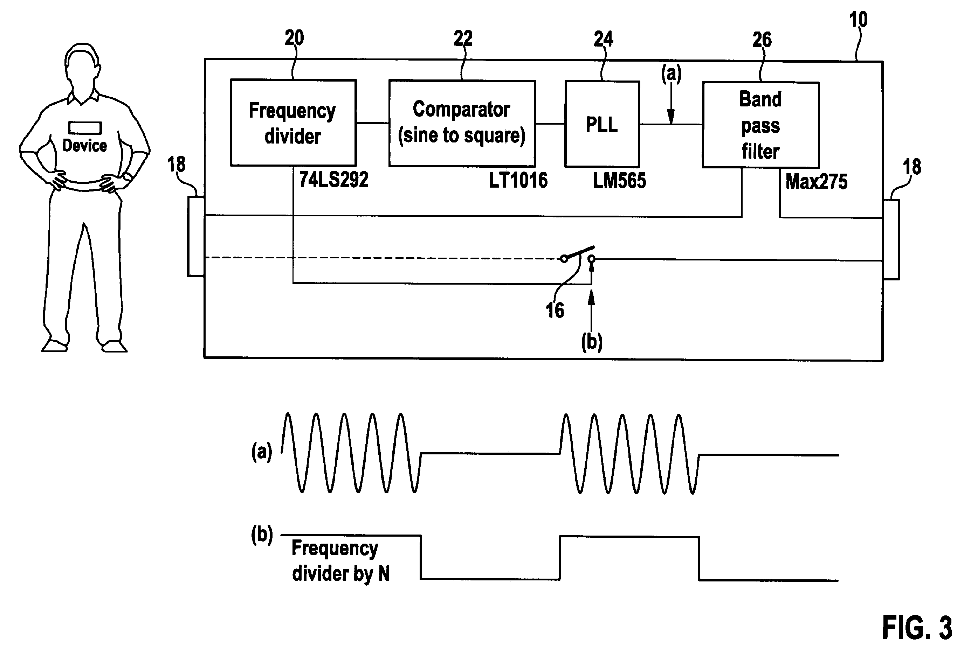

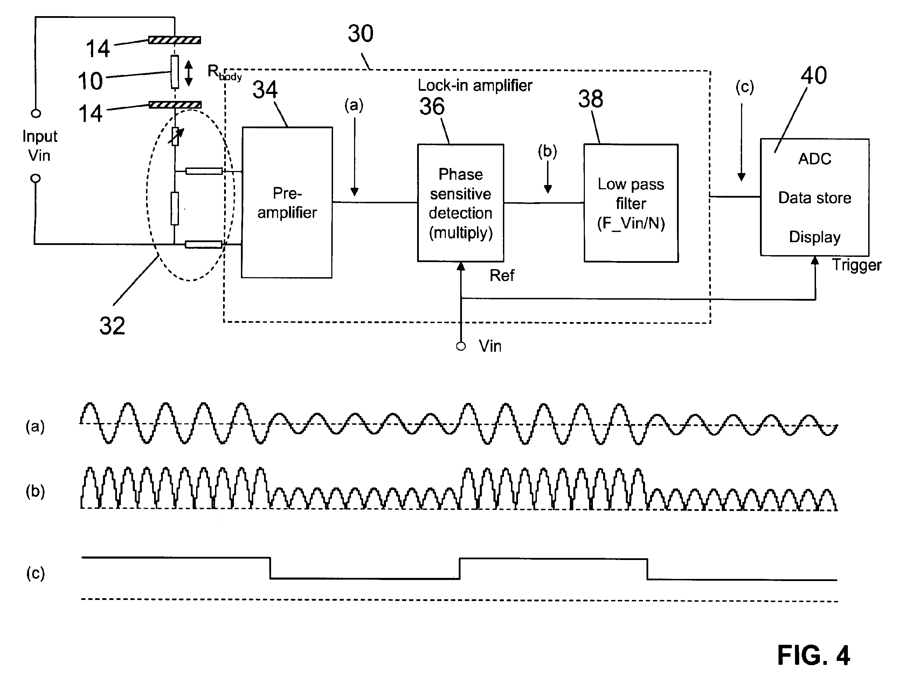

In the more detailed representations of the implantable medical device 10 and the external device 12 in figure 3 and 4, only those elements are depicted that are provided to implement the invention. It should be noted that a typical implantable medical device such as an implantable heart stimulator may further comprise a battery, a monitoring and/or therapy control unit, one or more therapy units such as stimulation units and a memory for storing control program and/or data sensed by the implantable medical device. If the implantable medical device is a pacemaker or an implantable cardioverter/defibrillator (ICD), the therapy units comprise stimulation units for generating and delivering electric stimulation pulses to a patient's heart tissue (myocardium). Stimulation units can be or are connected to stimulation electrode leads in a manner known per se.

In order to transmit data sensed by the implantable medical device to an external device a telemetry unit is provided. Typically the telemetry unit is configured to allow a bidirectional data communication, that is, the telemetry unit can transmit and receive data wirelessly. Figures 3 and 4 illustrate details of internal and external therapy units, respectively, according to the invention.

Referring to Fig. 3, the implantable medical device 10 comprises two (implantable) electrodes 18 that contact body tissue surrounding the implantable medical device 10 in its implanted state. Switching means diagrammatically represented by a simple switch 16 serve for connecting or disconnecting, respectively, the two electrodes 18 in order to establish or interrupt an electrical connection between the electrodes. The electrical connection across switching means 16 shall be of low ohmic resistance, sufficiently low to detectably decrease the volume impedance of the tissue surrounding the implanted medical device. The at least two electrodes 18 of the implantable medical device 10 preferably are arranged on the external surface of a hermetically sealed housing encapsulating the implantable medical device 10. According to a preferred embodiment, parts of the housing itself form the at least two electrodes 18.

In order to control switching means 16, the implantable medical device 10 further comprises a frequency divider 20 connected to a sine-to-square converting comparator 22 that in turn is connected to a phase-locked loop 24. Phase-locked loop 24 is connected to the electrodes 18 via band pass filter 26. Phase-locked loop 24 and frequency divider 20 are part of a switch control of implantable medical device 10.

The field induced between the cutaneous electrodes is sensed by the implantable medical device 10. The implantable medical device 10 locks in the frequency of the electric field using a phase-locked loop. Once the implantable medical device 10 is locked on to the frequency of the external device's induced field, it can activate the switch 16 between two electrodes 18 that are in the field (see figures 2 and 3) in synch with the frequency of the electric field.

In more detail: The implantable medical device 10 senses the imposed oscillatory electric signal as input signal that is detected via the electrodes 18 or across a resistor. Thus, the implantable medical device has an input sine signal that can be detected as an alternating voltage across electrodes 18 or across a resistor. This input sine signal is band pass filtered by band pass filter 26. A representation of that band pass filtered signal is shown in Fig. 3(a).

The band pass filtered input sine signal is fed to the phase-locked loop (PLL) 24 that locks in the frequency of the input sine signal. Phase-locked loop 24 puts out a synchronized sine signal to a comparator 22 that converts the sine signal so a square signal. The square signal thus generated is fed to frequency divider 20 that generates a clock for switching the switching means 16. The clock thus generated has frequency corresponding to a fraction of the frequency of the oscillatory electric field wherein the fraction is determined by a frequency division factor applied by frequency divider 20.

Alternatively, means to control switching 16 of the implantable medical device 10 may be connected to a pulse modulator which is not synchronized to the external electric field. For sufficiently low switching frequencies relative to the external field oscillatory frequency, for example 1:100 frequency ratio, the impact of lack of external field phase synchrony to the impedance modulation caused by switching is low. Only 1% sensitivity reduction of the external devices ability to detect the impedance changes induced by the implantable medical device would be caused by lack of phase synchrony for this example ratio.

The actual switching of switching means 16 further depends on data that are to be transmitted from the implantable medical device 10 to the external device 12. This data to be transmitted is coded and the code determines the actual sequence of switching of switching means 16.

Frequency divider 20 can be realized as a flip-flop counter. Switching means 16 shall have a small on-resistance (preferably less than the volume impedance of tissue in which the device is implanted), which leads to change of impedance of the tissue volume containing the implanted medical device, depending on whether switching means 16 are opened or closed.

Such change of impedance can be sensed by external device 12.

Data transmission from the implantable medical device to the external device thus can be summarized as follows:
Apply signal (oscillatory electric field) → propagate in body → switch on/off switching means in implantable device → impedance change of body → detect change by external device.

In the phase-synchronized embodiment, the switch control of the implantable medical device 10 thus receives an input sine signal by detecting a voltage across electrodes 18 or across a resistor. The switch control of the implantable medical device 10 according to the embodiment of figure 3 comprises band pass filter 26, phase-locked loop 24 to lock in the frequency of the input sine signal, comparator 22 to convert the sine signal to a square signal, a flip-flop counter that acts as frequency divider 20 that control switching means 16. Switching means 16 has a small on-resistance.

In the free-running switch controlled embodiment, the switch control of the implantable medical device 10 controls switching means 16 opening and closing without regard to the phase of the external electrical field. Switching means 16 again shall have a small on-resistance relative to the tissue volume impedance in which it is implanted.

The impedance changes caused by the implantable medical device 10 can be detected by the external device 12. As shown in Fig. 4, the external device 12 essentially comprises a lock-in amplifier that generates an output signal (signal c) that represents the signal transmitted by implantable medical device 10 by way of impedance changes. Lock-in amplifier 30 uses the signal imposed on a body by means of cutaneous electrodes 14 as a reference signal. For this purpose, a network of resistors 32 is provided that causes a voltage drop representing the signal (the oscillatory electric field) imposed on a body via cutaneous electrodes 14.

This signal is amplified by pre-amplifier 34 of lock-in amplifier 30.

The amplified signal sensed via cutaneous electrodes 14 and the resistor network 32 is fed to an AM demodulator comprising phase-sensitive detector 36 and further fed to a low pass filter 38, as depicted in Fig. 4. The amplified input signal sensed via cutaneous electrodes 14 and the resistor network 32 is represented as signal (a) in Fig. 4. The output signal of the phase-sensitive detector 36 is depicted in Fig. 4 as signal (b). The low pass filtered output signal of lock-in amplifier 30 is depicted in Fig. 4 as signal (c). Signal (c) corresponds to the signal generated by implantable medical device 10 and thus represents data to be transmitted from implantable medical device 10 to external device 12. This signal can be analog-to-digital converted and stored. Block 40 in Fig. 4 represents an analog-to-digital converter (ADC), a memory for data storage and a display of external device 12.

For the detection of impedance changes of body caused by the implantable medical device 10 the external device 12 thus comprises a lock-in amplifier that is configured to use the input as reference signal, comprising an AM demodulator which in turn comprises a precision rectifier and a low-pass filter. The low pass filtered signal is fed to an analog-to-digital converter or level discriminator in order to digitize the received signal. Due to the sensitivity of the phase locked demodulator (lock-in amplifier), the impedance changes detected may be very small, on the order of -120dB.

The communication from implantable medical device 10 to external device 12 thus can be understood as follows.

As the implantable medical device 10 alternatively shorts and opens the connection between the electrodes 18, the impedance between the external device electrodes 14 is slightly changed or modulated. The external device 12 can sense the change in impedance by measuring how much voltage or current is imparted on the electrodes 14 to create the oscillator electric field between the external device electrodes 14.

The external device 12 does this using a lock-in amplifier that is synchronized to the electric field frequency and phase. As the implantable medical device 10 modulates the impedance between the external device electrodes 14, the external device 12 integrates the changing current or voltage. The integration allows a very small change in sourced voltage or current to be detected using amplitude modulation.

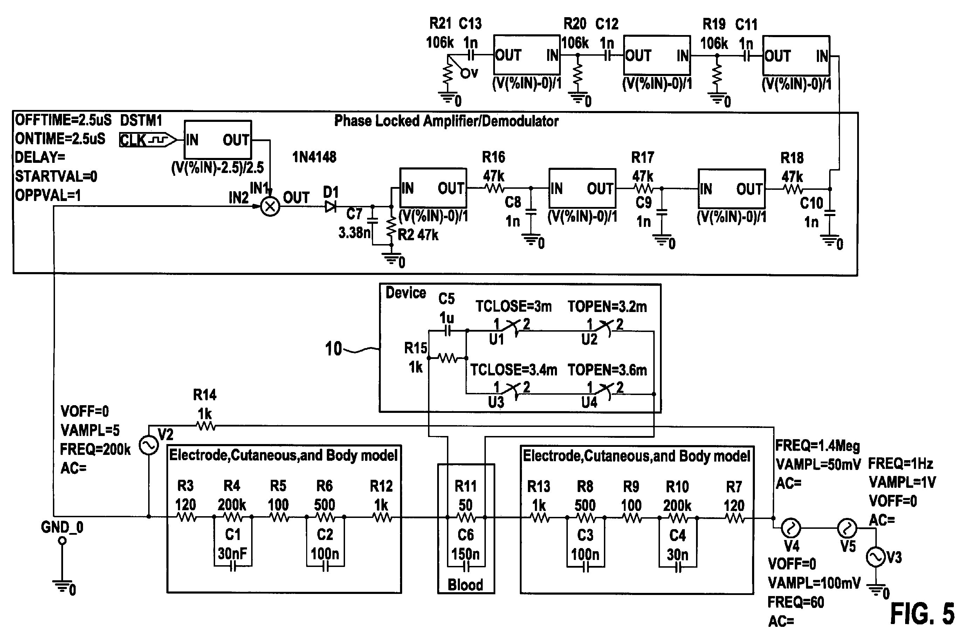

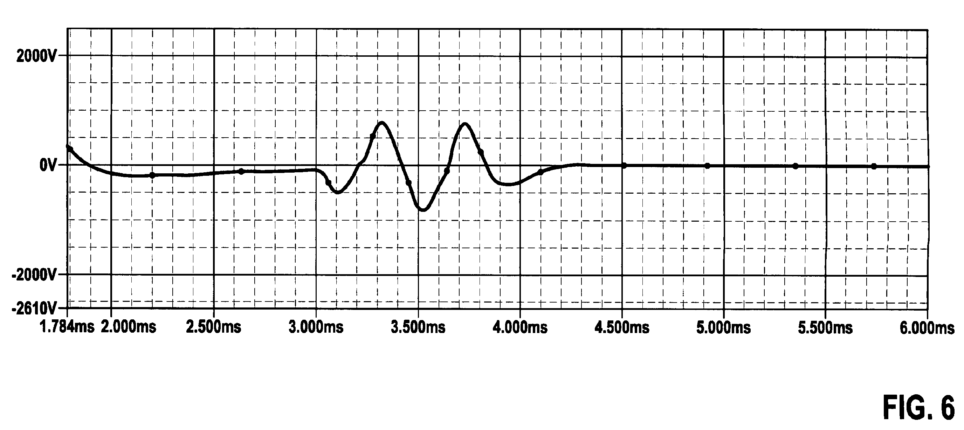

Communication can occur at approximately 1/10 to 1/1000th of the modulation frequency of the oscillatory electric field. This allows for ~ 10-1000 cycles of the electric field to be integrated to determine the imparted current or voltage. A model and simulation of the passive z-com embodiment is shown in figures 5 and 6.

In an alternative embodiment, a small electric field is generated by the implanted device in place of passive resistance changes. In more detail, an external device 12 generates a first oscillating electric field by means of cutaneous electrodes 14. The implanted device 10 detects this first oscillating electric field by its electrode means 18 and acquires a phase lock to the first oscillating electric field by means of a phase locked loop. The externally induced first oscillatory electric field may briefly be turned off by the external device while the phase locked loop maintains its phase information. The implanted device then generates a second oscillatory electric field by means of electrodes, the field having a fixed phase relationship with the first oscillatory electric field. The external device 12 detects and demodulates this second oscillatory electric field by cutaneous electrode means 14 and a phase-locked demodulator. Changing amplitude, phase, or other common modulation techniques may be used by the implanted medical device to modulate the second oscillatory electric field, thus comprising a communications means to transmit data to the external device.

In another embodiment, the implanted device switches its impedance or small electric field at every other crest of the external device's applied electric field for ~10-1000 cycles, called a chirp, in order to represent a mark of the communications scheme.

A communication from external device 12 to implantable medical device 10 can be done as follows:

The imposed oscillatory electric field has a fundamental frequency that the implantable medical device 10 uses to lock onto. The fundamental frequency is also used as a carrier frequency to send modulated data to the implantable medical device 10. The external device 12 modulates data, using frequency modulation or amplitude modulation, on top of the carrier imparted electric field. The implantable medical device 10 decodes the modulated data sensed through the electrodes.

The present invention does not require the implanted medical device to actively transmit data using its own power in the case of impedance modulation embodiment. It simply modulates a field imparted on it by an external device. In the embodiment where the device does emit a low amplitude electric field, the phase-locked property that it maintains allows for lower amplitude to be used for transmission to the external device than if simple bandlimited transmission schemes were used. In both embodiments, the resulting drain on the medical device's battery is extremely small. Because of the small power consumption, it is possible to transmit more data to the external device.

Figs. 5 and 6 illustrate a simulation of passive impedance-based data communication.

Standard electrode-electrolyte-body impedance models and blood impedance models were combined with a lock-in demodulator in a model of the passive impedance implementation of the invention. Each simulation component is labeled, with the 'device' modeled in box labeled with "10" with access impedance and four closing switches to simulate the communication of two data bits to the external receiver/demodulator. Environmental and breathing noise is induced in the model with voltage sources V3, V4, and V5 simulating line noise, RF noise, and breathing, body changing impedance noise, respectively.

The simulation signal at output filter resistor R21 is shown as the green trace in figure 6. The impedance changes are at a detectable level (showing 2 bits) beyond the background noise which has been filtered out due to the lock-in receiver system. The slope to the left of this plot shows the final settling time of the filters.

## Claims

1. Implantable device (10) comprising data communication means, said data communication means including means for altering an oscillatory electric field imposed on body tissue surrounding the implantable device (10) when the implantable device (10) is in its implanted state, wherein the oscillatory electric field has a frequency within a range of approximately 100 kHz to approximately 1 MHz,
**characterized in that**
the data communication means include means for generating a second oscillatory electric field which is synchronized to the oscillatory electric field imposed on body tissue surrounding the implantable device (10), wherein the means for generating the second oscillatory electric field comprise means for sensing the phase of the oscillatory electric field imposed on body tissue surrounding the implantable device (10), and means for generating the second oscillatory electric field in a manner that is phase synchronized to the oscillatory electric field imposed on body tissue surrounding the implantable device (10) while the medical device is in its implanted state.

2. Implantable device (10) according to claim 1, wherein the means for altering an oscillatory electric field are configured to modulate an impedance of a volume of body tissue surrounding the implantable device (10) when the implantable device (10) is in its implanted state and within an oscillatory electric field.

3. Implantable device (10) according to claim 2, further comprising switching means (16) and at least two electrodes (18) that are connected to the switching means (16) so they can be electrically connected or disconnected, respectively, by the switching means, wherein the at least two electrodes (18) are configured and arranged to cause a change of impedance when the implantable device (10) is in its implanted state and the electrodes (18) are connected or disconnected, respectively, by means of the switching means (16).

4. Implantable device (10) according to claim 3, wherein the switching means (16) are connected to a switch control that is configured to sense an oscillatory electric field imposed on body tissue surrounding the implantable device (10) when the implantable device (10) is in its implanted state.

5. Implantable device (10) according to claim 4, wherein the switch control comprises a phase-locked loop (PLL) (24) and a frequency divider (20), wherein the phase-locked loop (24) is configured to lock in a frequency of an oscillatory electric field imposed on body tissue surrounding the implantable device (10) when the implantable device (10) is in its implanted state, and wherein the frequency divider (20) is connected to the phase-locked loop (24) and is configured to divide a frequency signal put out by the phase-locked loop (24).

6. Implantable device (10) according to claims 3 and 4, wherein the switch control is connected to the at least two electrodes (18) and is configured to sense an oscillatory electric field imposed on body tissue via the at least two electrodes (18).

7. Implantable device (10) according to claim 5, wherein the switch control comprises a band-pass filter (26), wherein the band-pass filter (26) filters a signal fed to the phase-locked loop (24).

8. Implantable device (10) according to claim 1 or 7, wherein the means for generating the second oscillatory electric field include a lock-in amplifier (30).

9. Implantable device according to claim 1, wherein the means for altering an oscillatory electric field comprise field generating means for generating an oscillatory electric field that is phase-synchronized with an oscillatory electric field imposed on body tissue surrounding the implantable device when the implantable device is in its implanted state.

10. Implantable device according to claim 9, further comprising field generation control means that are operatively connected to the field generating means and are configured to control the field generating means in response to an oscillatory electric field imposed on body tissue surrounding the implantable device when the implantable device is in its implanted state.

11. Implantable device (10) according to any one of claims 1 to 10, wherein the implantable device (10) is a medical therapy and/or monitoring device having a hermetically sealed housing.

12. Data communication system including an implantable device (10) according to any one of claims 1 to 11 and further comprising an external device (12) that comprises or can be connected to at least two cutaneous electrodes (14), wherein the external device (12) further comprises external field generating means that are configured to generate an oscillatory electric field to be transcutaneously imposed on a body via the at least two cutaneous electrodes (14), wherein the external device (12) further comprises means for sensing alterations of body impedance and/or an oscillatory electric field generated by the implantable device (10) when the implantable device (10) is in its implanted state.

13. Data communication system according to claim 12, wherein the external device (12) comprises a phase-locked demodulator for demodulating amplitude-modulated signals and an analog-to-digital converter, wherein the analog-to-digital converter is operatively connected to the AM demodulator and the lock-in amplifier (30), and wherein the analog-to-digital converter puts out a signal that represents a signal transmitted by the implantable device (10).

14. Method of communicating data from an implantable device (10) to an external device (12), said method comprising:
- imposing an oscillatory electric field in body tissue encompassing an implantable device (10),
- sensing the imposed oscillatory electric field by means of the implantable device (10),
- altering the oscillatory electric field by means of the implantable device (10) by generating a small oscillatory electric field by means of the implantable device (10), wherein the oscillatory electric field is substantially phase synchronized to the imposed oscillatory electric field,
- sensing the small oscillatory electric field generated by the implantable device (10), respectively, by means of an external device (12) having or being connected to at least two cutaneous electrodes (14), wherein the oscillatory electric field has a frequency within a range of approximately 100 kHz to approximately 1 MHz.

15. Method according to claim 14, wherein the step of altering the oscillatory electric field by means of the implantable device (10) is performed by means of at least two electrodes (18) operatively connected or being part of the implantable device (10) and switching means (16) operatively connected to the at least two electrodes (18), wherein the switching means (16) connect and disconnect the at least two electrodes (18) in an alternating manner according to a code representing data to be transmitted from the implantable device (10) to the external device (12), said alternating connecting and disconnecting causing a detectable change of impedance for the imposed oscillatory electric field.

16. Method according to claim 14 or 15, wherein the step of sensing the change of impedance comprises sensing a current or voltage over cutaneous electrodes (14) and integrating a sensed voltage or current over a number of cycles of the oscillatory electric field, said number of cycles corresponding to frequency division factor of a frequency divider (20) of the implantable device (10).

17. Method according to any one of claims 14 to 16, wherein the data communication includes data communication from an external device (12) to an implantable device (10), said method further comprising the step of modulating the oscillatory electric field by means of the external device (12).

## Patentansprüche

1. Implantierbare Einrichtung (10), die Datenübertragungsmittel umfasst, wobei die Datenübertragungsmittel Mittel zum Ändern eines schwingenden elektrischen Felds aufweisen, das in Körpergewebe eingeprägt wird, das die implantierbare Einrichtung (10) umgibt, wenn sich die implantierbare Einrichtung (10) im implantierten Zustand befindet, wobei das schwingende elektrische Feld eine Frequenz in einem Bereich von ungefähr 100 kHz bis ungefähr 1 MHz aufweist,
**dadurch gekennzeichnet, dass**
die Datenübertragungsmittel Mittel zum Erzeugen eines zweiten schwingenden elektrischen Felds aufweisen, das mit dem schwingenden elektrischen Feld synchronisiert ist, das in Körpergewebe eingeprägt wird, das die implantierbare Einrichtung (10) umgibt, wobei die Mittel zum Erzeugen des zweiten schwingenden elektrischen Felds Mittel zum Erfassen der Phase des schwingenden elektrischen Felds umfassen, das in Körpergewebe eingeprägt wird und das die implantierbare Einrichtung (10) umgibt, und Mittel zum derartigen Erzeugen des zweiten schwingenden elektrischen Felds, dass das zweite schwingende elektrische Feld es zum schwingenden elektrischen Feld phasensynchronisiert ist, das in Körpergewebe eingeprägt wird und das die implantierbare Einrichtung (10) umgibt, während sich die implantierbare Einrichtung (10) im implantierten Zustand befindet.

2. Implantierbare Einrichtung (10) nach Anspruch 1, wobei die Mittel zum Ändern eines schwingenden elektrischen Felds so eingerichtet sind, dass sie eine Impedanz eines Volumens von Körpergewebe moduliert, das die implantierbare Einrichtung (10) umgibt, wenn sich die implantierbare Einrichtung (10) im implantierten Zustand und innerhalb eines schwingenden elektrischen Felds befindet.

3. Implantierbare Einrichtung (10) nach Anspruch 2, die ferner Schaltmittel (16) und mindestens zwei Elektroden (18) umfasst, die mit den Schaltmitteln (16) verbunden sind, sodass sie durch die Schaltmittel elektrisch verbunden beziehungsweise getrennt werden können, wobei die mindestens zwei Elektroden (18) so eingerichtet und angeordnet sind, dass sie eine Impedanzänderung bewirken, wenn sich die implantierbare Einrichtung (10) im implantierten Zustand befindet und die Elektroden (18) mittels der Schaltmittel (16) verbunden beziehungsweise getrennt werden.

4. Implantierbare Einrichtung (10) nach Anspruch 3, wobei die Schaltmittel (16) mit einer Schaltsteuerung verbunden sind, die so eingerichtet ist, dass sie ein schwingendes elektrisches Feld erfasst, das in Körpergewebe eingeprägt wird, das die implantierbare Einrichtung (10) umgibt, wenn sich die implantierbare Einrichtung (10) im implantierten Zustand befindet.

5. Implantierbare Einrichtung (10) nach Anspruch 4, wobei die Schaltsteuerung eine Phasenregelschleife (PLL) (24) und einen Frequenzteiler (20) umfasst, wobei die Phasenregelschleife (24) so eingerichtet ist, dass sie bei einer Frequenz eines schwingenden elektrischen Felds einrastet, das in Körpergewebe eingeprägt wird, das die implantierbare Einrichtung (10) umgibt, wenn sich die implantierbare Einrichtung (10) im implantierten Zustand befindet, und wobei der Frequenzteiler (20) mit der Phasenregelschleife (24) verbunden und so eingerichtet ist, dass er ein Frequenzsignal teilt, das die Phasenregelschleife (24) ausgibt.

6. Implantierbare Einrichtung (10) nach Anspruch 3 und 4, wobei die Schaltsteuerung mit den mindestens zwei Elektroden (18) verbunden und so eingerichtet ist, dass sie ein schwingendes elektrisches Feld erfasst, das über die mindestens zwei Elektroden (18) in Körpergewebe eingeprägt wird.

7. Implantierbare Einrichtung (10) nach Anspruch 5, wobei die Schaltsteuerung ein Bandpassfilter (26) umfasst, wobei das Bandpassfilter (26) ein der Phasenregelschleife (24) zugeführtes Signal filtert.

8. Implantierbare Einrichtung (10) nach Anspruch 1 oder 7, wobei die Mittel zum Erzeugen des zweiten schwingenden elektrischen Felds einen Trägerfrequenzverstärker (30) aufweisen.

9. Implantierbare Einrichtung nach Anspruch 1, wobei die Mittel zum Ändern eines schwingenden elektrischen Felds Felderzeugungsmittel zum Erzeugen eines schwingenden elektrischen Felds umfassen, das zu einem schwingenden elektrischen Feld phasensynchronisiert ist, das in Körpergewebe eingeprägt wird, das die implantierbare Einrichtung umgibt, wenn die implantierbare Einrichtung im implantierten Zustand ist.

10. Implantierbare Einrichtung nach Anspruch 9, ferner umfassend Felderzeugungssteuerungsmittel, die funktionsmäßig mit den Felderzeugungsmitteln verbunden und so eingerichtet sind, dass sie die Felderzeugungsmittel als Reaktion auf ein schwingendes elektrisches Feld steuern, das in Körpergewebe eingeprägt wird, das die implantierbare Einrichtung umgibt, wenn die implantierbare Einrichtung im implantierten Zustand ist.

11. Implantierbare Einrichtung (10) nach einem der Ansprüche 1 bis 10, wobei die implantierbare Einrichtung (10) eine medizinische Behandlungs- und/oder Überwachungseinrichtung mit einem hermetisch verschlossenen Gehäuse ist.

12. Datenübertragungssystem, aufweisend eine implantierbare Einrichtung (10) nach einem der Ansprüche 1 bis 11 und ferner umfassend eine externe Einrichtung (12), die mindestens zwei Hautelektroden (14) umfasst oder damit verbunden sein kann, wobei die externe Einrichtung (12) ferner externe Felderzeugungsmittel umfasst, die so eingerichtet sind, dass sie ein schwingendes elektrisches Feld erzeugen, das über die mindestens zwei Hautelektroden (14) transkutan in einen Körper eingeprägt werden soll, wobei die externe Einrichtung (12) ferner Mittel zum Erfassen von Veränderungen der Körperimpedanz und/oder eines schwingenden elektrischen Felds umfasst, das von der implantierbaren Einrichtung (10) erzeugt wird, wenn die implantierbare Einrichtung (10) im implantierten Zustand ist.

13. Datenübertragungssystem nach Anspruch 12, wobei die externe Einrichtung (12) einen phasenstarren Demodulator zum Demodulieren von amplitudenmodulierten Signalen und einen Analog-Digital-Wandler umfasst, wobei der Analog-Digital-Wandler funktionsmäßig mit dem AM-Demodulator und dem Trägerfrequenzverstärker (30) verbunden ist und wobei der Analog-Digital-Wandler ein Signal ausgibt, das ein Signal darstellt, das die implantierbare Einrichtung (10) sendet.

14. Verfahren zum Übertragen von Daten von einer implantierbaren Einrichtung (10) an eine externe Einrichtung (12), wobei das Verfahren Folgendes umfasst:
- Einprägen eines schwingenden elektrischen Felds in Körpergewebe, das um eine implantierbare Einrichtung (10) herum angeordnet ist,
- Erfassen des eingeprägten schwingenden elektrischen Felds mittels der implantierbaren Einrichtung (10),
- Ändern des schwingenden elektrischen Felds mittels der implantierbaren Einrichtung (10) durch Erzeugen eines kleinen schwingenden elektrischen Felds mittels der implantierbaren Einrichtung (10), wobei das schwingende elektrische Feld zu dem eingeprägten schwingenden elektrischen Feld im Wesentlichen phasensynchronisiert ist,
- Erfassen des kleinen schwingenden elektrischen Felds, das von der implantierbaren Einrichtung (10) beziehungsweise mittels einer externen Einrichtung (12) erzeugt wird, die mindestens zwei Hautelektroden (14) aufweist oder damit verbunden ist, wobei das oszillatorische elektrische Feld eine Frequenz in einem Bereich von ungefähr 100 kHz bis ungefähr 1 MHz aufweist.

15. Verfahren nach Anspruch 14, wobei der Schritt des Änderns des schwingenden elektrischen Felds mittels der implantierbaren Einrichtung (10) mithilfe von mindestens zwei Elektroden (18), die funktionsmäßig mit der implantierbaren Einrichtung (10) verbunden oder Bestandteil davon sind, und Schaltmitteln (16) durchgeführt wird, die funktionsmäßig mit den mindestens zwei Elektroden (18) verbunden sind, wobei die Schaltmittel (16) die mindestens zwei Elektroden (18) abwechselnd entsprechend einem Code verbinden und trennen, der Daten darstellt, die von der implantierbaren Einrichtung (10) an die externe Einrichtung (12) übertragen werden sollen, wobei das abwechselnde Verbinden und Trennen eine nachweisbare Impedanzänderung für das eingeprägte schwingende elektrische Feld bewirkt.

16. Verfahren nach Anspruch 14 oder 15, wobei der Schritt des Erfassens der Impedanzänderung ein Erfassen eines Stroms oder einer Spannung über Hautelektroden (14) und ein Integrieren einer erfassten Spannung oder eines erfassten Stroms über mehrere Zyklen des schwingenden elektrischen Felds umfasst, wobei die Zyklenzahl einem Frequenzteilungsfaktor eines Frequenzteilers (20) der implantierbaren Einrichtung (10) entspricht.

17. Verfahren nach einem der Ansprüche 14 bis 16, wobei die Datenübertragung eine Datenübertragung von einer externen Einrichtung (12) an eine implantierbare Einrichtung (10) umfasst, wobei das Verfahren ferner den Schritt des Modulierens des schwingenden elektrischen Felds mittels der externen Einrichtung (12) umfasst.

## Revendications

1. Dispositif implantable (10) comprenant des moyens de communication de données, lesdits moyens de communication des données incluant des moyens pour la modification d'un champ électrique oscillant imposé à un tissu humain entourant le dispositif implantable (10) lorsque le dispositif implantable (10) est dans son état implanté, où le champ électrique oscillant a une fréquence dans une gamme d'approximativement 100 kHz jusqu'à 1 MHz approximativement,
**caractérisé en ce que**
les moyens de communication des données incluent des moyens pour la génération d'un second champ électrique oscillant qui est synchronisé avec le champ électrique oscillant imposé au tissu humain entourant le dispositif implantable (10), où les moyens pour la génération du second champ électrique oscillant comprennent des moyens pour la détection de la phase du champ électrique oscillant imposé au tissu humain entourant le dispositif implantable (10), et des moyens pour la génération du second champ électrique oscillant d'une manière qui est synchronisée en phase avec le champ électrique oscillant imposé au tissu humain entourant le dispositif implantable (10) alors que le dispositif médical est dans son état implanté.

2. Dispositif implantable (10) selon la revendication 1, dans lequel les moyens pour la modification du champ électrique oscillant sont configurés pour moduler une impédance d'un volume de tissu humain entourant le dispositif implantable (10) lorsque le dispositif implantable (10) est dans son état implanté et dans un champ électrique oscillant.

3. Dispositif implantable (10) selon la revendication 2, comprenant en outre des moyens de commutation (16) et au moins deux électrodes (18) qui sont reliées aux moyens de commutation (16) de sorte qu'elles peuvent être connectées ou déconnectées électriquement, respectivement, par les moyens de commutation, où les au moins deux électrodes (18) sont configurées et disposées pour provoquer un changement d'impédance lorsque le dispositif implantable (10) est dans son état implanté et les électrodes (18) sont connectées ou déconnectées, respectivement, à l'aide des moyens de commutation (16).

4. Dispositif implantable (10) selon la revendication 3, dans lequel les moyens de commutation (16) sont reliés à une commande de commutation qui est configurée pour détecter un champ électrique oscillant imposé au tissu humain entourant le dispositif implantable (10) lorsque le dispositif implantable (10) est dans son état implanté.

5. Dispositif implantable (10) selon la revendication 4, dans lequel la commande de commutation comprend une boucle à asservissement de phase (PLL) (24) et un diviseur de fréquence (20), où la boucle à asservissement de phase (24) est configurée pour se verrouiller à une fréquence du champ électrique oscillant imposé au tissu humain entourant le dispositif implantable (10) lorsque le dispositif implantable (10) est dans son état implanté, et où le diviseur de fréquence (20) est relié à la boucle à asservissement de phase (24) et est configuré pour diviser un signal de fréquence sortant par la boucle à asservissement de phase (24).

6. Dispositif implantable (10) selon les revendications 3 et 4, dans lequel la commande de commutation est reliée aux au moins deux électrodes (18) et est configurée pour détecter un champ électrique oscillant imposé au tissu humain par l'intermédiaire des au moins deux électrodes (18).

7. Dispositif implantable (10) selon la revendication 5, dans lequel la commande de commutation comprend un filtre passe-bande (26), dans lequel le filtre passe-bande filtre le signal d'alimentation de la boucle à asservissement de phase (24).

8. Dispositif implantable (10) selon les revendications 1 ou 7, dans lequel les moyens pour la génération du second champ électrique oscillant incluent un amplificateur à verrouillage (30).

9. Dispositif implantable selon la revendication 1, dans lequel les moyens pour la modification du champ électrique oscillant comprennent des moyens pour la génération d'un champ électrique oscillant qui est en synchronisation de phase avec un champ électrique oscillant imposé au tissu humain entourant le dispositif implantable lorsque le dispositif implantable est dans son état implanté.

10. Dispositif implantable selon la revendication 9, comprenant en outre des moyens de commande de la génération du champ qui sont reliés de manière fonctionnelle au champ générant les moyens et sont configurés pour commander les moyens de génération du champ en réponse à un champ électrique oscillant imposé au tissu humain entourant le dispositif implantable lorsque le dispositif implantable est dans son état implanté.

11. Dispositif implantable (10) selon l'une quelconque des revendications 1 à 10, dans lequel le dispositif implantable (10) est un dispositif de surveillance et/ou de traitement médical qui a un boîtier hermétiquement scellé.

12. Système de communication des données incluant un dispositif implantable (10) selon l'une quelconque des revendications 1 à 11 et comprenant en outre un dispositif externe (12) qui comprend ou peut être relié aux au moins deux électrodes cutanées (14), dans lequel le dispositif externe (12) comprend en outre des moyens de génération d'un champ externe qui sont configurés pour générer un champ électrique oscillant destiné à être imposé de manière transcutanée à un corps par l'intermédiaire des au moins deux électrodes cutanées (14), où le dispositif externe (12) comprend en outre des moyens pour la détection des modifications de l'impédance du corps et/ou du champ électrique oscillant généré par le dispositif implantable (10) lorsque le dispositif implantable (10) est dans son état implanté.

13. Système de communication des données selon la revendication 12, dans lequel le dispositif externe (12) comprend un démodulateur à asservissement de phase pour démoduler les signaux modulés en amplitude et un convertisseur analogique numérique, où le convertisseur analogique numérique est relié de façon fonctionnelle au démodulateur AM et à l'amplificateur à verrouillage (30), et où le convertisseur analogique numérique délivre un signal qui représente un signal transmis par le dispositif implantable (10).

14. Procédé de communication de données en provenance d'un dispositif implantable (10) vers un dispositif externe (12), ledit procédé comprenant :
- l'application d'un champ électrique oscillant à un tissu humain entourant un dispositif implantable (10),
- la détection du champ électrique oscillant imposé au moyen du dispositif implantable (10),
- la modification du champ électrique oscillant au moyen du dispositif implantable (10) par la génération d'un petit champ électrique oscillant au moyen du dispositif implantable (10), où le champ électrique oscillant est substantiellement en synchronisation de phase avec le champ électrique oscillant imposé,
- la détection du petit champ électrique oscillant généré par le dispositif implantable (10), respectivement, au moyen du dispositif externe (12) qui a été, ou qui est connecté aux au moins deux électrodes cutanées (14), où le champ électrique oscillant a une fréquence dans une gamme d'approximativement 100 kHz jusqu'à 1 MHz approximativement.

15. Procédé selon la revendication 14, dans lequel l'étape de modification du champ électrique oscillant au moyen du dispositif implantable (10) est réalisée au moyen des au moins deux électrodes (18) reliées de façon fonctionnelle ou faisant partie du dispositif implantable (10) et des moyens de commutation (16) reliés de façon fonctionnelle aux au moins deux électrodes (18), où les moyens de commutation (16) connectent et déconnectent les au moins deux électrodes (18) de manière alternée selon un code représentant les données à transmettre à partir du dispositif implantable (10) en direction du dispositif externe (12), lesdites connexion et déconnexion alternées provoquant un changement d'impédance détectable par le champ électrique oscillant imposé.

16. Procédé selon les revendications 14 ou 15, dans lequel l'étape de détection du changement d'impédance comprend la détection d'un courant ou d'une tension sur les électrodes cutanées (14) et l'intégration de la tension ou du courant détecté(e) sur un nombre de cycles du champ électrique oscillant, ledit nombre de cycles correspondant au facteur de division de la fréquence du diviseur de fréquence (20) du dispositif implantable (10).

17. Procédé selon l'une quelconque des revendications 14 à 16, dans lequel la communication des données inclut la communication des données en provenance d'un dispositif externe (12) vers un dispositif implantable (10), ledit procédé comprenant en outre l'étape de modulation du champ électrique oscillant au moyen du dispositif externe (12).
